(19) Europäisches Patentamt
European Patent Office
Office européen des brevets

(11) **EP 0 668 066 B1**

(12) **EUROPEAN PATENT SPECIFICATION**

(45) Date of publication and mention
of the grant of the patent:
**21.07.1999 Bulletin 1999/29**

(51) Int. Cl.⁶: **A61F 13/15, A61F 13/50**

(21) Application number: **95102285.4**

(22) Date of filing: **17.02.1995**

(54) **Body conforming absorbent article**

An den Körper anschmiegender absorbierender Artikel

Article absorbant conformant au corps

(84) Designated Contracting States:
**AT BE CH DE ES FR GB GR IE IT LI NL PT SE**
Designated Extension States:
**SI**

(30) Priority: **18.02.1994 US 198809**

(43) Date of publication of application:
**23.08.1995 Bulletin 1995/34**

(73) Proprietor: **McNEIL-PPC, INC.**
**Milltown New Jersey 08850 (US)**

(72) Inventors:
• **McCoy, Sherlyn S.**
**Skillman New Jersey 08558 (US)**
• **Mavinkurve, Pramod**
**Edison, NJ 08820 (US)**

(74) Representative:
**Groening, Hans Wilhelm, Dipl.-Ing. et al**
**BOEHMERT & BOEHMERT**
**Franz-Joseph-Strasse 38**
**80801 München (DE)**

(56) References cited:
EP-A- 0 304 957      EP-A- 0 335 252
EP-A- 0 359 501      EP-A- 0 605 017
WO-A-92/10984        WO-A-93/09745
US-A- 4 433 972

• DATABASE WPI Section Ch, Week 9302 Derwent
Publications Ltd., London, GB; Class D22, AN
93-013430 & JP-A-04 341 261 (KAO) , 27
November 1992

**Description**

Background of the Invention

1. Field of the Invention

This invention relates to an absorbent article in accordance with the preamble of claim 1 for absorbing body fluids such as menstrual fluid, vaginal discharge and/or urine. More particularly, this invention relates to an absorbent article having preferential bending zones which enable the article to conform closely to the body in order to maintain contact with the body and reduce the probability of leakage and failure. The preamble of claim 1 is based on EP-A-0 304 957.

2. Prior Art

EP-A-0 304 957 discloses a sanitary napkin having a fluid retarding means disposed transversely across its absorbent element for inhibiting the transmission of body fluid from a central portion of the absorbent element to one of the elements transverse ends. In order to effect additional protection from staining at the ends of the sanitary napkin, hydrophobic material may be located at the transverse ends of the absorbent pad. The hydrophobic material acts in combination with the cross channels to provide additional protection against failure at the ends of the pad. The cross channels resist or delay the absorption of fluid at the ends of the pad. The hydrophobic material provides a fluid repellant barrier, which prevents the fluid from passing into the ends of the pad and, by contacting the fluid, prevents it from leaking out to the wearer's panty. The design is especially suited to winged napkin configurations, especially those that have absorbent tissues in their flaps or wings. A preferred configuration includes two channels which are placed across the width of the absorbent pad such that the ends of each channel are within the portion of the napkin protected by the flaps. The channels preferably represent compressed portions of the absorbent element which allow the transmission of body fluid after a central portion of the absorbent element is saturated. The channels provide a better fitting napkin, since they represent a point at which the pad may be readily bent to conform to the body of the wearer. The ends of the preferred napkin can raise up with less resistance than that of a conventional napkin and, thus, provide a closer fit. This improved fit, particularly at the pad ends leads to improved protection.

WO-A-9 210 984 discloses a sanitary napkin in Fig. 1 featuring a liquid pervious topsheet, a liquid impervious backsheet, and an absorbent core intermediate the topsheet and the backsheet. The napkin has a caliper of less than 4 mm and prefereably less than about 6 mm may have two flaps extending from each longitudinal side margin of the napkin. The core is divided into a plurality of independent segments. The segments are transversely independently segmented. They are considered to be "independent" if the segments may have relative movement in the Z-direction without being constrained from such movement by an adjacent segment. A 3-segment core of which the first of the three regions is thought of as the mons region having a compound curved convex upward shape. The second region is defined by the labia majora and resembles a W-shaped outline. The third region is determined by the gluteal groove and is generally cup-shaped and defined by two convex upwardly and outwardly diverging lines. Furthermore, the napkin may have three disconnected segments wherein said segments are indirectly connected, such as through topsheet, backsheet or any tissue wrapping the core.

Database WPI, Section Ch, Week 9302, Class 22, An 93-013430 & JP-A-4 341 261 discloses a sanitary towel having first and second flexible shafts formed in central long axis part in width direction to enable folding of said napkin.

US-A-4 433 972 discloses a sanitary napkin comprising a backing sheet and a face sheet having an absorbent pad assembly sandwiched therebetween. The pad assembly includes two pads, there being a relatively large pad of wood fluff or like absorbent material, and a substantially diamond-shaped second pad of lesser dimensions seated on said first pad for transmitting fluid thereto. The second pad is impregnated with a germicide.

WO-A-9 309 745 discloses an absorbent article comprising a liquid permeable topsheet, a liquid impermeable backsheet, a liquid absorbent pad and a liquid distribution strip. The liquid distribution strip is positioned between the topsheet and the absorbent pad. The liquid distribution strip comprises a nonwoven web which has a screen pattern having high fiber density portions with reduced spacing between the fibers and/or an embossed pattern having a highly compressed portion. The high fiber density portion of the screen pattern is oriented primarily to the longitudinal ends of the absorbent pad so as to distribute the liquid exuded to the longitudinal ends. The highly compressed portions of the embossed pattern are oriented primarly to the longitudinal ends so as to inhibit the distribution of liquid toward the lateral edges.

Conventional full-size sanitary protection and feminine hygiene products such as adult incontinence devices typically contain an absorbent element, a fluid-pervious body-contacting element and a fluid-impervious undergarment-facing element. The principle function of such articles is to absorb body fluid from the wearer and retain the fluid in order to prevent the fluid from soiling the wearer's garments. It is well-known that conventional feminine hygiene articles do not adequately fulfill women's protection requirements. Theoretically, sanitary napkins have the capability of absorbing between 50 and 100 grams of fluid. However, soiling still occurs even when only 5 to 10 grams of fluid is deposited on the absorbent articles. One of the primary reasons why soiling occurs is that conventional napkins fit poorly to women's bodies. When there is a space between the user and the pad, the fluid tends to travel along the contours of the body, resulting in soiling of the undergarment.

One factor that contributes to poor coverage and poor fit is lack of napkin stability. When a compression force is applied to a napkin (e.g., by the user's thighs), the napkin tends to fold or bunch resulting in a smaller area of coverage. The napkin center may also be depressed (i.e., move away from the user's body). Fluid then travels along the body and bypasses the napkin leading to soiling of the undergarment.

An additional factor that contributes to poor fit centers on the mistaken belief that the outer genital area of females, especially in the areas of the urethral and vaginal openings, is curved when in fact it is essentially flat or planar. Thus, there is a tendency on the part of some inventors to focus on a curved product, wrongly assuming that the centermost portions of such a product will fit closely to the body.

Several prior art patents discuss absorbent systems which attempt to address the soiling problem in different ways. One method described in a number of patents is to create a resilient and/or stabilized absorbent system in order to prevent the napkin from bunching when worn. For example, U.S. Patent No. 4,195,634 (DiSalvo) describes a "stiffener means" which is incorporated into the napkin and positioned between the absorbent core and the barrier along the entire length of the pad to resist side compression. However, this design is deficient in that the stiffening/stabilizing element is below the absorbent medium. The absorbent medium tends to collapse when exposed to fluid, causing it to move away from the wearer, despite the presence of the stiffener means. Furthermore, the stiffener means, being nonconformable, does not fit the napkin to the body and is quite uncomfortable because it spans the length of the absorbent, keeping the entire napkin in a planar configuration. The design described in U.S. Patent No. 4,405,326 (Lenggham) is similarly deficient. U.S. Patent No. 4,217,901 (Bradstreet) also describes a crush-resistant sanitary napkin, but does not specifically address the issue of body-fit.

European Patent Applications 0 335 252 and 0 335 253 (Buell) describe a disposable absorbent article having a flexure-resistance, deformation element that is not moisture-sensitive. The deformation element has a convex upward configuration when the napkin is worn and pressed inward by the thighs. The proposed design relies on the lateral compressive forces of the wearer's thighs to form a convex upward configuration. However, this design is also flawed with respect to maintaining body-fit and conformation. The convex configuration cannot consistently be controlled by thigh movement, as the napkin's ability to conform to the body relies heavily upon product placement and the wearer's anatomy. Furthermore, there is no means to insure that the product will stay in place at the appropriate portion of the anatomy. In addition, if the deformation element is not moisture-sensitive, the deformation element cannot be placed near the pad's surface because it cannot absorb fluid. Thus, the absorbent material in the pad which is closer to the body than the deformation element will tend to collapse when exposed to fluid, and move away from the body.

Some prior art patents address the problems of bunching and absorbent collapse by suggesting an increase in the thickness of the central portion of the absorbent element. Some suggest interlabial products that have a portion fitting between the labia, close to the vaginal orifice. For example, U.S. Patent No. 4,490,147 (Pierce) describes a sanitary napkin having a raised center with absorbent pads which are arranged parallel to one another in a pyramid-shaped bundle. The pads are movable with respect to one another and are encased by a liquid pervious cover material. However, such a napkin will not conform to the perineal cavity, particularly at the front and back. The pyramid shape may also cause fluid to roll off the napkin's edge and cause soiling of the wearer's undergarment.

U.S. Patents Nos. 4,631,062 and 4,804,380 (Lassen) generally describe self-conforming napkins for partial labial disposition. The napkin contains a posterior region, including a raised profile for placement intermediate the wearer's labia majora and a flattened front portion for placement exterior of the clitoris and pubic mons. However, these products would tend to move during wear and can easily become dislodged from the vestibule. Furthermore, interlabial napkins tend to be uncomfortable to wear for most women.

Other raised center napkin designs are described in U.S. Patent No. 2,662,527 (Jacks). In Jacks, the absorbent pad has a main body member of absorbent material and a second absorbent material secured on the face of the main member. The second absorbent material is narrower and shorter than the main member and has dimensions designed to allow it to fit between the labia. Such a design would tend to collapse when exposed to fluid and would be extremely likely to move in relation to the body of the wearer.

Similarly, the napkin described in U.S. Patent No. 3,406,689 (Hicks) has a dual discrete layer system in which the two pads are separated from one another and are freely movable with respect to each other. The two pads are installed separately, would tend to move and are extremely inconvenient. Fluid may also travel from one pad to the edge of the other, resulting in staining the wearer's undergarment. U.S. Patent No. 4,433,972 (Malfitano) also contains two pads, although the top pad is hourglass in shape.

U.S. Patent No. 4,425,130 (Desmarais) describes a sanitary napkin having a primary menstrual absorbent pad and a "panty protector" which are joined. Such a design has the same types of deficiencies as those described above with regard to compound pads. For example, the panty protector member is intended to protect the user's garments from being soiled by fluids which are expelled from the primary menstrual pad or which inadvertently pass the primary pad. However, this design may encourage fluid to wick to the edge of the panty protector and transfer to the user's garment. It is also possible for the panty protector to fold in and contact the face of the primary menstrual pad. Fluid may then transfer to the user's panty if the panty folds up and around the edges of the panty protector, especially if the panty protector is thin.

Another multicomponent system is described in patent publication No. WO 92/07535 (Visscher and Osborne). According to this publication, a liquid pervious spacing structure moves the top body-facing cover sheet away from the absorbent core of the napkin. The spacing structure has an uncompressed and a compressed configuration and an upper and lower portion. However, the components of this construction are unlikely to remain separated when exposed to fluid, due to wet-collapse.

Other types of prior art products which are results of attempts to conform napkins to the body are curved sanitary napkins. For example, European Patent No. 091,412 depicts an absorbent product having an absorbent core which is convex in the center and has elasticized side margins. U.S. Patent No. 4,770,657 (Ellis) also describes a curved sanitary napkin with elasticized side edges.

Another example of a curved sanitary napkin is illustrated in WO 88/04547 (Thoren) which discloses a design in which elastic means are prestretched. This causes the napkin to be resiliently distorted so that the sides assume a convex shape when held against the user's body. Upon application of pressure, the shape will flatten out. However, when the pressure is reduced or eliminated, the napkin resumes its curved shape. Further this design lacks a structural element and, therefore, when wetted, will collapse and move away from the user and cause soiling of the undergarment.

Other products which are designed to protect undergarments from staining with body fluid are those having side extensions that can wrap around a panty crotch. Examples of such products are described in U.S. Patent No. 3,397,697 (Rickard), U.S. Patent No. 4,285,343 (McNair), U.S. Patents Nos. 4,589,876 and 4,687,478 (Van Tilburg) and EP Publication No. 0426235 (Osborne). Yet other examples of such designs are described in U.S. Patent No. 4,608,407 (Mattingly), U.S. Patent No. 4,911,701 (Mavinkurve) and U.S. Patent No. 4,900,320 (McCoy). However, many of these products do not fit to the body and may still result in staining due to wicking along the body around the side extensions and onto the user's thighs and garments or wicking where the side extension is not present.

Thus, it is an object of this invention to create a sanitary napkin and/or adult incontinence product capable of conforming to the body and capturing fluid as it leaves the body.

It is another object of this invention to provide an absorbent product which can protect the user's undergarment by providing a large area of coverage.

It is a further object of this invention to provide a comfortable and discreet means for absorbing body fluid.

Yet another object of this invention is to provide an absorbent product which is resilient, resistant to wet collapse, yet highly absorbent.

## SUMMARY OF THE INVENTION

The invention achieves the afore-mentioned objects by the features as disclosed in claim 1. Accordingly, this invention relates to an absorbent product having longitudinal sides and transverse ends, a body-facing surface and a garment-facing surface. The absorbent product of this invention contains:

   a) a fluid-permeable cover on the body-facing surface;
   b) a fluid-absorbent core adjacent the fluid-permeable cover, the fluid-absorbent core having a central portion located inward of the transverse ends, the fluid-absorbent core further containing at least one preferential bending zone transverse to the longitudinal axis of the article; and
   c) a fluid-impermeable barrier on the garment-facing surface.

Preferably, the fluid-absorbent core contains two preferential bending zones. The fluid-absorbent core further preferably contains a structural zone in the center portion between the two preferential bending zones. The structural zone preferably contains a resilient absorbent material capable of resisting collapse when exposed to fluid.

When placed in an undergarment, the absorbent products of this invention will display a resilient portion adjacent the labia majora and the vaginal orifice and will preferentially bend along the preferential bending zones when subjected to the forces of the undergarment raised against the body. This provides a snug fit against the body along the front and back of the user while the central portion provides an intimate fit against the labia majora. The structural zone provides resilience and resistance to wet-collapse in the area where it is needed, i.e., near the vaginal orifice or urethra, where the body fluid exits the user and impinges on the absorbent product.

Thus, the products of this invention provide superior leakage protection due to better body fit without sacrificing comfort.

The absorbent products of this invention optionally, but preferably, contain attachment tabs along the longitudinal sides of the absorbent core. These attachment tabs are preferably of dimensions such that their longitudinal edges are shorter than the longitudinal dimensions of the structural zone so as not to impede the product's bending along the preferential bending zones located adjacent the structural zone.

BRIEF DESCRIPTION OF THE DRAWINGS

Figure 1 shows the absorbent article of this invention in plan view from the body-facing side of the article.

Figure 2 is a cross-sectional view of the absorbent article shown in Figure 1 taken along the line 2-2.

Figure 3 is a cross-sectional view of the absorbent article shown in Figure 1 taken along the line 3-3.

Figure 4 shows a custom jig used in determining x-directional deformation.

DETAILED DESCRIPTION OF THE PREFERRED EMBODIMENTS

The products **5** of this invention preferably have the following elements:

a) a fluid-permeable cover **10** on the body-facing surface **20** of the article,

b) a fluid-impermeable backsheet **30**,

c) an absorbent core **40** containing

(1) at least one preferential bending zone **50**, and

(2) a center portion **60** containing a structural zone **70**.

Fluid-permeable cover sheet

The fluid-permeable cover sheet **10** may be composed of a woven or non-woven fibrous fabric or an apertured plastic sheet. The fibrous fabric may be composed of cellulosic fibers such as cotton or rayon, or it may be composed of polyolefin fibers such as polyethylene. It may be an entangled or modified-entangled fabric or spun-bond or melt-blown fabric.

Apertured plastic covers are well-known in the art. Additional fluid-permeable cover sheets from the prior art are absorbent, porous, dry-laid, nonwoven webs or scrim type materials such as those described by I.S. Ness in U.S. Pat. No. 4,880,419 and by Campau in U.S. Pat. No. 3,044,467, Hendricks in U.S. Pat. No. 3,463,154 and Sneider in U.S. Pat. No. 3,570,491 (herein incorporated by reference).

There are also fluid-permeable cover sheets of the prior art such as those described by T. J. Luceri in U.S. Pat. No. 4,795,455, by S. Cadieux in E.P. Pat. No. 0 359,501, by A. T. Mays in E.P. Pat. No. 70,163, and by R. P. James in U.S. Pat. No. 4,368,323 (herein incorporated by reference) which are nonwovens made from hydrophobic fibers which have been coated with an adhesive or have been subjected to heat and/or pressure to fuse the individual fibers to each other. Such materials tend to have only limited absorption but serve to allow passage to lower layers for absorption and retention. As a result, the fluid is wicked away from the body, leaving the surface of the body-contacting layer feeling drier to the touch.

The use of hydrophobic fibers for the body contacting, or cover, layer allows fluid to pass through to the absorbent layers beneath yet will not retain moisture on the surface layer, thus providing greater comfort to the wearer by feeling dry for a longer period of time. The desirability of such a feature has been recognized by Levesque U.S. Pat. No. 3,838,692 (herein incorporated by reference) who describes a chemical method of providing porosity to hydrophobic materials.

Fluid-impermeable backsheet

The fluid-impermeable backsheet **30** may be a nonwoven or woven fabric treated to become impervious to fluid. Typically, though, the backsheet **30** is a plastic sheet composed of polyethylene or polypropylene. Such layers are taught, for example in U.S. Pat. No. 4,731,066 (Korpman).

Absorbent core

The absorbent core **40** is, typically, a rectangular or hourglass shaped pad that is separate from, possibly integral with, the cover. The absorbent core **40** is preferably made from wood pulp or other cellulosic material. Such cores are taught, for example, in U.S. patents no. 4,552,618 (Kopolow) and 4,536,432 (Holtman) and in British patent no. 2,189705 (Mesek), incorporated herein by reference.

Additionally, the absorbent core **40** may utilize a variety of fluid immobilizing materials, e.g., superabsorbing polymers or peat moss, as a reservoir layer to increase fluid capacity or minimize pad bulk. The reservoir layer may be made from cellulosic materials and, additionally, such materials as sphagnum peat moss and superabsorbent polymers which, pound for pound, absorb much greater quantities of fluid than pulp alone, allowing for the manufacture of much thinner absorbent pads. Typically, materials such as sphagnum and superabsorbent polymer are not used alone, but in conjunction with cellulosic pulp in order to provide extra absorbency for heavy fluid flow and to provide bulk to beep the product closer to the user's body. Such materials are taught by Y. Levesque in U.S. Patent No. 4,507,122; S. Dabi in U.S. Pat. No. 4,944,963; by I. S. Hess in U.S. Pat. No. 4,880,419; by J. Roller in U.S. Pat. No. 4,443,492; hereby incorporated by reference.

When such fluid immobilizing materials are used, however, it has been found that while they have the ability to absorb many times their weight in body fluid, the rate of absorption is relatively slow. Thus, it is often preferred that a transfer layer **80** be incorporated into the absorbent core **40**. The transfer layer **80** functions to draw fluid from the fluid-permeable cover sheet **10** and transport it to that portion of the core into which the bulk of the fluid will eventually be absorbed, often called the reservoir layer **90**. Thus, placement of the transfer layer **80** in the absorbent product would be between the cover sheet **10** and the reservoir layer **90**. Typically, a transfer layer **80** functions to quickly absorb fluid and hold it until the slower absorbing reservoir layer **90** can accept it. Acceptable transfer layers **80** are those made from cellulosic materials, such as wood pulp, and an adhesive like binder. The basis weight of such materials would range from about 20-200 g/m$^2$. More preferably, they would range from about 50-150 g/m$^2$. Still more preferably, they would range from about 75-100 g/m$^2$.

Structural zone

The absorbent core **40** contains a central portion **60** which is located near the center of product **5**, which is defined by the portion between the longitudinal edges **100** and approximately midway between the transverse edges **110**. The central portion **60** would then be positioned, when in use, closest to the point where fluid exits the body. Preferentially, this central portion **60** contains a structural zone **120** that is slightly raised above the plane defined by the longitudinal edges **100** to keep in close contact with the body, is absorbent, and maintains its shape in the wet state.

The structural zone **120** is intended to maintain contact between the absorbent product **5** and the body at that point where fluid exits the body. The structural zone **120** should be fluid-absorbent and resistant to wet-collapse. The resistance to wet-collapse enables the structural zone **120** to better maintain contact with the body. Body-contact is important because it helps to inhibit expressed fluid from travelling along the body, e.g, the user's thighs, to soil the user's clothes. Fluid migration along the body is a key element of soiling. Fluid migration generally occurs when fluid is not immediately absorbed into an absorbent product. Thus, the closer and longer the absorbent product **5** can maintain contact with the body, the less likely that the fluid will leak or migrate away from the absorbent product and cause soiling.

The structural zone **120** of the absorbent core **40** may consist of a single layer that is merely a continuation of the absorbent core **40** itself wherein additional pulp, for example, is simply added to the core to raise it up. Such a layer of pulp may be added between the absorbent core **40** and cover **10**. However, it is preferred to add it between the absorbent core **40** and the fluid impervious back sheet **30**. This configuration will raise the transfer layer/peat moss combination (for example) closer to the body.

Preferably, the length of the structural zone **120** should conform generally to the dimensions of the labia majora so as to cover the area from which fluid will exit the body. Thus, it should be between about 50.8 and 177.8 mm (2 and about 7 inches) in length. More preferably, the structural zone **120** should be between about 76.2 and 152.4 mm (3 and about 6 inches) in length. The length of the structural zone **120** should be less than that of the product **5** as a whole. Preferably, it should be less than about 80% of the product **5** length and, more preferably, between about 25-50% of the product **5** length. The structural zone **120** may preferably be between about 25.4 and 76.2 mm (1 and about 3 inches) in width. The structural zone **120** should be resistant to wet-collapse in both the X-direction (herein defined as that direction transverse to the longitudinal edges **100**) and the Z-direction (herein defined as the direction normal to the cover sheet **10**). The wet collapse in the Z-direction should be less than 10% at $1.38 \cdot 10^3$ Pa (0.2 psi) and less than 25% at 3.45 · 10$^3$ Pa (0.5 psi). It should have a deformation measurement of less than 15% in the dry state and less than 35% in the wet state in accordance with the Wet and Dry Deformation Tests described in Example 1 below.

Preferential bending zones

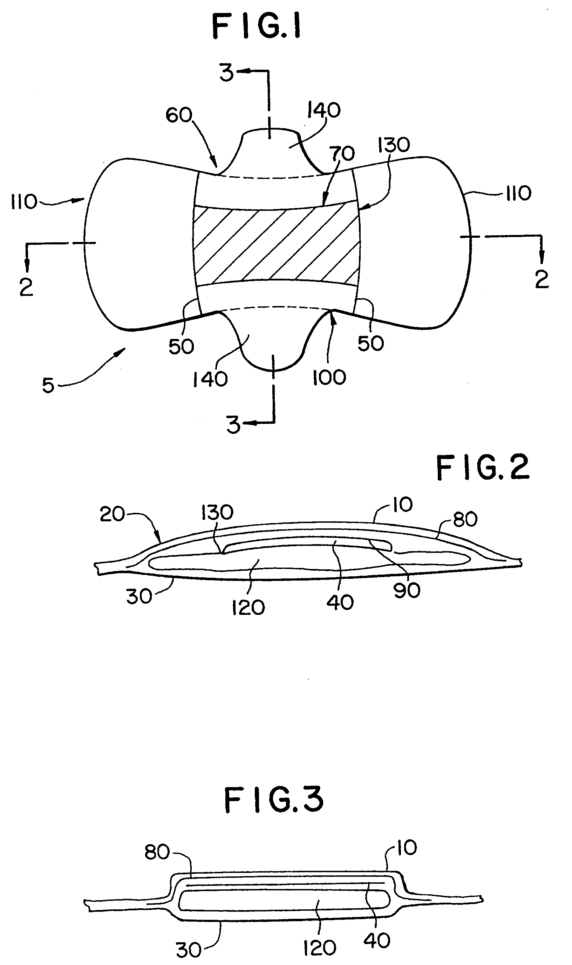

The bending zone(s) **50** must be in a region outside the structural zone **120**. Preferentially, the bending zone(s) **50** is (are) located at the end(s) **130** of the structural zone **120**. This allows the product **5** to cup to the front and back of the user while maintaining an essentially planar or flat configuration (when viewing the longitudinal edges of the product **5** from a longitudinal cross section - see for example Figure 2), thus allowing the structural zone **120** in the central portion **60** of the article **5** to conform to the shape of the user's body. Most preferred are bending zones **50** which are transverse to the longitudinal axis of the absorbent article **5** and extend from one longitudinal side **100** to the other.

The bending location and profile of the napkin **5** should be such that the central portion **60** maintains an almost linear profile when the ends of the napkin **5** are deflected by a specified amount. Example 3 below describes the testing for characterization of such preferential bending zones **50**. It is undesirable to have the central portion **60** cup or curve as the napkin **5** would move away from the user and protection would be sacrificed. The bending zone **50** may occur by a change in the flexibility of the napkin **5** along its length or it may occur due to a seam, space or embossment anywhere in those portions of the napkin **5** adjacent the structural zone **120**. An additional method of forming the preferential bending zones **50** involves changing the density of the napkin **5** such that the density of the structural zone **120** is much greater than that of the napkin near the transverse ends **110**. Changing the density may occur by changing the thickness of the napkin **5** in the z-direction or by changing the basis weights of the materials in those parts of the napkin **5** adjacent the central portion **60**.

The napkin **5** may also be pre-shaped in such a way that the napkin **5**, prior to use, has a bend located at the preferential bending zones. Such pre-shaping may be accomplished, for example by placing elastic in the flanged side margins of longitudinal edges **100** of the napkin **5**.

In a preferred embodiment, where the absorbent core **40** contains a sphagnum element coextensive with the structural zone **120**, the bending zones **50** are at each end of the structural zone **120** because there are natural hinge points at the end of the sphagnum element.

Attachment tabs **140** may be used to hold the article **5** securely in place so that the structural zone **120** maintains intimate contact with the body. The attachment tabs **140** also serves to prevent the user's panty from contacting the longitudinal sides **100** of the central portion **60** by restraining the elastic of the panty. It is preferred, however, that the line of juncture of the attachment tabs **140** not extend beyond the bending zones **50** as this would inhibit bending of the article **5**. Attachment tabs **140** may be attached to the longitudinal sides **100** of the article **5** or they may be located on the garment facing side inward from the longitudinal edges, e.g., U.S. patent no. 4,900,320 (McCoy).

It is preferable that the line of juncture between the tabs **140** and the napkin **5** be less than 177.8 mm (7 inches). It is more preferable that the line of juncture be between 76.2 and 152.4 mm (3 and 6 inches,).

It is also preferred that the absorbent article **5** be hourglass shaped with the front and rear portions wider than the central portion **60**. This configuration will also help to stabilize the article **5** by inhibiting shifting from front to back.

In order to demonstrate the features of this invention, the examples below are submitted. In all instances the napkins manufactured for testing in the examples consisted of a perforated plastic film (PE/EVA or PE) for the cover (either 18.13 or 33.91 g/m$^2$ (0.54 or 1.0 oz/yd$^2$), a transfer layer of 90 g/m$^2$ airlaid, latex bonded pulp fabric, a wetlaid cellulosic absorbent having a basis weight of 325 g/m$^2$, a core of wood pulp fluff underneath (garment side) of a sphagnum absorbent board, and a PE/EVA film barrier layer. Bending zones were created by embossing transversely across the samples at each end of the structural zone (defined as the ends of the sphagnum board) and by decreasing the thickness of the pads both fore and aft of the structural zone.

Example 1 - Z-direction Deformation Test

The test described below was designed to simulate the z-direction pressure exerted on a sanitary napkin by a wearer's body. Ideally, the napkin should not collapse in the z-direction when exposed to body fluids, such as menstrual fluid, and body pressure. As demonstrated below, conventional napkins are subject to such collapse.

Ten napkins were prepared according to the teachings of the specification and ten control napkins, commercially available from Proctor & Gamble under the trademark ALWAYS PLUS MAXI, were purchased. Each sample was conditioned at 21.11 ± 1.11 °C (70° + 2° F) and 50 ± 2% RH for 24 hours prior to the test. Such conditions were maintained throughout the testing. The test is further described below. The discussion will detail how each sample was tested.

In addition to the napkin samples, synthetic menstrual fluid (SMF) was prepared by dissolving 0.15% poly acrylamide in isotonic phosphate buffer. Approximately 0.3% Germabean was added to prevent bacterial growth. An amount of the pH of the fluid was measured to 7.4 and the viscosity at 30 cps at 1 radian per second.

An AMES gauge #91-013, with a 28.575 mm (1.125 inch) diameter foot weighing 12.7 grams, was calibrated using standard blocks under a standard 56.7 gram weight (the total weight corresponds to a pressure of $1.034 \cdot 10^3$ Pa (0.15 psi)). The foot of the gauge was raised and the sample was placed on the anvil. The foot was then lowered gently onto the center of the sample and allowed to remain for 15 seconds before taking a reading. This is the initial thickness $W_1$.

The weight on the AMES gauge was then increased to 85.3 grams (this plus the weight of the foot corresponds to a pressure of $1.52 \cdot 10^3$ Pa (0.22 psi)). The thickness of the sample was again measured and noted as thickness $W_2$.

The center of the sample was marked where the foot of the gauge was resting. The sample was removed and 10cc of SMF was introduced to the marked area using a syringe. The fluid was added slowly enough so that it did not spill outside the marked area. The sample was then immediately placed on the AMES gauge with a weight of 85.3 grams. The thickness of the sample was noted then and for each minute thereafter until there was no change in the thickness of the sample. (In all samples, the thickness ceased to change after 4 minutes). This final thickness was recorded as $W_3$.

All pressure was then removed from the sample by removing it from the AMES gauge, and it was allowed to stand for 15 minutes. After 15 minutes the sample was placed in the AMES gauge and a weight of 56.7 grams was introduced. The thickness under this pressure was recorded as $W_4$.

From these four readings it is possible to calculate the % reduction in thickness normal use (%RT) by the following equation:

$$\%RT = (W_2 - W_3)/W_2 \times 100$$

From these four readings it is also possible to calculate the % delayed reduction in thickness (%DR) by the following equation:

$$\%DR = (W_1 - W_4)/W_1 \times 100$$

Ten additional napkins were prepared according to the teachings of the specification and an additional ten control napkins, commercially available from Procter & Gamble under the trademark ALWAYS PLUS MAXI, were purchased. Each sample was tested according to the procedure described above except that the test pressure, i.e., that recorded as $W_2$, was done using a 226.8 gram weight (corresponding to a total pressure of $3.65 \cdot 10^3$ Pa (0.53 psi)).

Table 1 shows the results obtained under a test pressure of $1.52 \cdot 10^3$ Pa (0.22 psi). Table 2 shows the results obtained under a test pressure of $3.65 \cdot 10^3$ Pa (0.53 psi).

TABLE 1

| TEST PRESSURE AT $1.52 \cdot 10^3$ Pa (0.22 PSI) | | | | |
|---|---|---|---|---|
| | Test | | Control | |
| | %RT | %DR | %RT | %DR |
| | 7.496 | 4.210 | 30.769 | 30.320 |
| | 7.192 | 5.980 | 29.725 | 28.012 |
| | 3.580 | -1.300 | 26.375 | 26.179 |
| | 5.187 | -1.408 | 29.566 | 23.994 |
| | 5.650 | 1.284 | 24.296 | 21.407 |
| | 3.571 | -3.102 | 24.160 | 23.566 |
| | 10.069 | 4.232 | 23.414 | 24.606 |
| | 6.666 | 0.160 | 24.437 | 23.557 |
| | 4.566 | -2.215 | 23.455 | 22.606 |
| | 6.593 | -0.356 | 23.411 | 22.186 |
| Mean | 6.057 | 0.747 | 25.961 | 24.643 |
| S.D. | 1.988 | 3.090 | 2.945 | 2.776 |

TABLE 2

| TEST PRESSURE AT $3.65 \cdot 10^3$ Pa (0.53 PSI) | | | | |
|---|---|---|---|---|
| | Test | | Control | |
| | %RT | %DR | %RT | %DR |
| | 24.750 | 23.279 | 36.538 | 32.132 |
| | 26.615 | 22.684 | 37.681 | 31.288 |
| | 28.740 | 27.632 | 36.501 | 35.453 |
| | 27.490 | 25.333 | 34.904 | 34.407 |
| | 26.556 | 27.682 | 35.088 | 27.190 |
| | 23.390 | 23.611 | 38.021 | 37.215 |
| | 19.408 | 17.139 | 36.678 | 38.587 |
| | 21.839 | 17.949 | 37.343 | 29.566 |
| | 18.868 | 15.120 | 35.528 | 34.153 |
| | 22.901 | 20.886 | 39.082 | 34.894 |
| Mean | 24.056 | 22.131 | 36.736 | 33.489 |
| S.D. | 3.370 | 4.324 | 1.335 | 3.476 |

Example 2 - X-direction Deformation Test

The objective of this test is to determine the deformation resistance of a sanitary napkin or any absorbent article in X-direction (the direction transverse to the longitudinal axis across the plane of the napkin) in terms of a loss in napkin width in both wet and dry states. Generally the tests consists of holding a napkin around the inner cylinder of an Instron deformation test apparatus. It is compressed and then allowed to recover from its deformation for 2 cycles. The loss in width as a result of compression and relaxation is a measure of the deformation resistance of the napkin in X-direction.

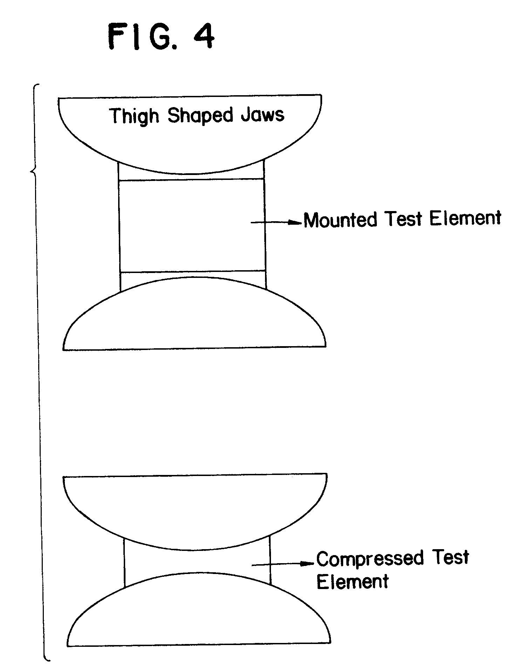

The apparatus used for the test was: 1. Instron Model 1122 Universal Tester; 2. a custom jig (Figure 4) with an 203.2 mm (8 inch) diameter cylinder and upper and lower jaws which are shaped to resemble a human thigh; 3. 100 Kg load cell; 4. vernier caliper; 5. 25 $cm^3$ (cc) graduated cylinder; 6. plexiglas plate (0.511 thick) with an oblong center opening of 19.05 mm x 38.1 mm (0.75" x 1.50"); 7. a 76.2 mm (3 inch) wide knitted fabric (45% polyester/45% cotton/10% Lycra spandex) which simulates an undergarment crotch; 8. SMF prepared in the manner described in Example 1 above.

Ten napkins were prepared according to the teachings of the specification and ten control napkins, commercially available from Procter & Gamble under the trademark ALWAYS MAXI PLUS, were purchased. Each sample was conditioned at 21.11°C ± 1.11°C (70° ± 2° F) and 50 ± 2% RH for 24 hours prior to the test. Such conditions were maintained throughout the testing. The test is further described below. The discussion will detail how each sample was tested.

The gap between the upper and lower compression surfaces of the Custom Jig was set at 20.32 mm (0.8 inch). The return limit on the Instron console was set at 55.88 mm (2.2 inches). The crosshead speed was set at 127 mm (5 in.)/minute. The test direction was set to "up" and was pressed so that the crosshead starts moving, stopping when reaching a 76.2 mm (3 inch) gap spacing (20.32 mm (0.8 Inch) initial gap + 55.88 mm (2.2 inch)crosshead travel).

The instrument was then calibrated by setting the load scale dial to 2 Kg and zeroing the pen on the chart. A 1 Kg weight was then placed on the lower cylinder ring jaw and pen was adjusted using the "calibration knob" so that the pen rested on the 1 Kg line. The I Kg weight was then removed. If the pen returns back to the zero position on the chart, it means the instrument has been calibrated. If needed, the "balance knob" is used to bring the pen back to zero.

The 76.2 mm (3 inch) wide fabric was cut such that the grain of the fabric was perpendicular to the direction of travel. A 101.6 mm (4 inch) long test sample element was cut from the center of each sanitary napkin to be tested. The width of each test sample was the width of the absorbent system in the napkin. The width of the test element was measured using a Vernier caliper and was recorded as initial width $L_1$.

The release paper, which is on the garment facing side, was removed from the sample. The sample was then placed with the garment facing side down into the center of the inner surface of the loop to be formed on the Jig with the side edges of the fabric enveloping the sample edges. The sample was secured on the fixture in such a way that it was in direct contact with the jig surface and centered between the curved jaws.

The cycle counter was set to "2" so that the test sample could be compressed for 2 cycles. The return limit on the console was set to "2.25." The maximum and minimum limits were set to "2.20" and "0.00" respectively. The test direction was set to "down", and the crosshead speed was set to 127 mm (5 in.)/minute.

The Instron was started. After the crosshead had compressed the sample for 2 cycles and stopped, the sample was is removed and the width again measured and is recorded as final width $L_2$.

Napkins were also tested in the wet state by preparing the samples as described above. However, prior to positioning the sample on the jig, 10cc of SMF was applied to the center of the test element. This was done by placing the plexiglas plate over the center of the sample. The 10 $cm^3$(cc) of fluid was poured from the graduated cylinder into the oblong opening of the plate placed on to the sample. Once the fluid was absorbed, the sample was allowed to remain at test conditions for 1 minute and then $L_1$ and $L_2$ measured as described above.

The percentage of amount of x-direction deformation (%XD) was calculated as follows:

$$\%XD = (L_1 - L_2)/L_1 \times 100$$

Table 3 shows the results obtained in the dry state. Table 4 shows the results obtained in the wet state.

TABLE 3

| % LOSS IN WIDTH IN X-DIRECTION IN DRY STATE | | | | | |
|---|---|---|---|---|---|
| TEST | | | CONTROL | | |
| L1 | L2 | %XD | L1 | L2 | %XD |
| 50.55 (1.99) | 43.18 (1.70) | 14.57 | 41.91 (1.65 | 36.58 (1.44) | 12.73 |
| 48.01 (1.89) | 40.89 (1.61) | 14.81 | 43.69 (1.72) | 34.54 (1.36) | 20.93 |
| 48.51 (1.91) | 43.43 (1.71) | 10.47 | 44.45 (1.75) | 33.02 (1.30) | 26.47 |
| 49.78 (1.96) | 44.20 (1.74) | 11.22 | 43.69 (1.72) | 35.56 (1.40) | 18.60 |
| 48.51 (1.91) | 45.21 (1.78) | 6.80 | 43.18 (1.70) | 37.85 (1.49) | 13.53 |
| 46.48 (1.83) | 40.64 (1.60) | 12.57 | 45.21 (1.78) | 35.31 (1.39) | 21.91 |
| 46.74 (1.84) | 41.91 (1.65) | 10.33 | 43.43 (1.71) | 34.04 (1.34) | 21.64 |
| 45.97 (1.81) | 42.67 (1.68) | 7.18 | 44.20 (1.74) | 37.08 (1.46) | 16.09 |
| 45.48 (1.83) | 41.91 (1.65) | 9.84 | 41.91 (1.65) | 35.81 (1.41) | 14.54 |
| 46.23 (1.82) | 41.91 (1.65) | 9.34 | 44.20 (1.74) | 37.08 (1.46) | 16.09 |
| Mean = | | 10.71 | | | 18.24 |
| S.D. = | | 2.71 | | | 4.42 |

TABLE 4

| % LOSS IN WIDTH IN X-DIRECTION IN WET STATE | | | | | |
|---|---|---|---|---|---|
| TEST | | | CONTROL | | |
| L1 | L2 | %XD | L1 | L2 | %XD |
| 47.24 (1.86) | 32.26 (1.27) | 31.72 | 42.93 (1.69) | 24.89 (0.98) | 42.01 |
| 46.24 (1.84) | 31.24 (1.23) | 33.15 | 42.93 (1.69) | 23.62 (0.93) | 44.97 |
| 48.26 (1.90) | 33.53 (1.32) | 30.53 | 41.91 (1.65) | 25.15 (0.99) | 40.00 |

TABLE 4 (continued)

| % LOSS IN WIDTH IN X-DIRECTION IN WET STATE | | | | | |
|---|---|---|---|---|---|
| TEST | | | CONTROL | | |
| L1 | L2 | %XD | L1 | L2 | %XD |
| 46.48 (1.83) | 32.51 (1.28) | 30.05 | 42.42 (1.67) | 24.89 (0.98) | 41.32 |
| 47.50 (1.87) | 33.78 (1.33) | 28.88 | 42.67 (1.68) | 26.92 (1.06) | 36.90 |
| 45.72 (1.80) | 32.00 (1.26) | 30.00 | 41.66 (1.64) | 25.4 (1.00) | 39.02 |
| 46.99 (1.85) | 32.51 (1.28) | 30.81 | 41.66 (1.64) | 22.1 (0.87) | 46.95 |
| 45.21 (1.78) | 31.24 (1.23) | 30.90 | 42.16 (1.66) | 23.62 (0.93) | 43.98 |
| 46.48 (1.83) | 32.51 (1.28) | 30.05 | 42.42 (1.67) | 23.88 (0.94) | 43.71 |
| 46.48 (1.83) | 31.24 (1.23) | 32.79 | 40.89 (1.61) | 23.62 (0.93) | 42.24 |
| Mean = | | 30.89 | | | 42.11 |
| S.D. = | | 1.32 | | | 2.97 |

As is clear from both Table 3 and Table 4, the napkin as described in this invention offers more resistance to deformation in X-direction as compared to conventional napkins. The conventional napkins, which mainly consist of pulp, undergo deformation in X-direction in both dry as well as wet states. This phenomenon, called bunching, reduces the effective surface area of the absorbent system available for the capture of bodily fluid. The severe deformation in X-direction, therefore, potentially can lead to higher chances of undergarment staining.

## Claims

1.  An absorbent article (5) having longitudinal sides (100), transverse ends (110), a longitudinal axis, a body-facing surface (20) and a garment facing surface (30), said article comprising:

    (a) a fluid-permeable cover (10) on said body-facing surface;
    (b) a fluid-absorbent core (40) adjacent said fluid-permeable cover (10), said fluid-absorbent core (40) having a central portion (60) located inward of said transverse ends (110) and having a resilient structural zone which is resistant to wet collapse,
    (c) said fluid-absorbent core (40) further comprising two preferential bending zones (50), the preferential bending zones (50) being transverse to the longitudinal axis and wherein the structural zone is located between the preferential bending zones; and
    (d) a fluid-impermeable barrier (30) on said garment-facing surface, wherein
    (e) said absorbent article (5) preferentially bends at said preferential bending zone (50)
    **characterized in that**
    (f) the preferred bending zone (50) is produced by changing the density of the absorbent article (5) such that the central portion (60) of the absorbent article (5) comprises a material having a basis weight which is greater than that of the materials near the transverse ends (110), and wherein said structural zone (120) is conformed to the perineal area adapted to cover labia majore.

2.  The absorbent article of claim 1 wherein the central portion (60) maintains an essentially planar configuration in use.

3.  The absorbent article of claim 1 wherein the absorbent core (40) contains a transfer layer (80) and a reservoir layer (90).

4.  The absorbent article of claim 3 wherein the reservoir layer (90) is sphagnum.

5.  The absorbent article of claim 1 wherein the absorbent article (5) contains attachment tabs (130).

6.  The absorbent article of claim 5 wherein the attachment tabs (130) are affixed to the absorbent article (5) at positions between each preferential bending zone (50).

7. The absorbent article of claim 1 wherein the structural zone (120) being resistant to wet collapse in both, the X-direction defined as the direction transverse to the longitudinal edges (100) and the Z-direction defined as the direction normal to the cover sheet (10), whereby the wet collapse in the Z-direction should be less than 10% at 1.38 . $10^3$ Pa (0.2 psi) and less than 25% at 3.45 . $10^3$ Pa (0.5 psi) .

8. The absorbent article of claim 1 wherein the distance between said bending zones (60) is between 76.2 and 152.4 mm (3 and 6 inches).

**Patentansprüche**

1. Absorbierender Gegenstand in (5) mit längs verlaufenden Seiten (100), querliegenden Enden (110), einer Längsachse, einer dem Körper Zugewandten Fläche (20) und einer der Wäsche zugewandten Fläche (30), wobei der Gegenstand aufweist:

   (a) eine flüssigkeitsdurchlässige Abdeckung (10) auf der dem Körper zugewandten Fläche;
   (b) einen flüssigkeitsabsorbierenden Kern (40) angrenzend an die flüssigkeitsdurchlässige Abdeckung (10), wobei der flüssigkeitsabsorbierende Kern (40) einen mittleren Teil (60) hat, der sich innerhalb der querliegenden Enden (110) befindet und eine nachgiebige strukturelle Zone hat, die gegen das Zusammenfallen bei Nässe widerstandsfähig ist,
   (c) wobei der flüssigkeitsabsorbierende Kern (40) weiterhin zwei bevorzugte Biegezonen (50) aufweist, wobei die bevorzugten Biegezonen (50) quer zur Längsachse verlaufen und wobei die strukturelle Zone zwischen den bevorzugten Biegezonen angeordnet ist; und
   (d) eine flüssigkeitsundurchlässige Barriere (30) auf der der Unterwäsche zugewandten Fläche, wobei
   (e) sich der absorbierende Gegenstand (5) bevorzugt an den bevorzugten Biegezonen (50) biegt,
   **dadurch gekennzeichnet, daß**
   (f) die bevorzugte Biegezone (50) durch die Änderung der Dichte des absorbierenden Gegenstandes (5) erzeugt wird, derart, daß der mittlere Teil (60) des absorbierenden Gegenstandes (5) ein Material aufweist, das ein Flächengewicht hat, welches größer ist als das des Materials nahe den querliegenden Enden (110) und wobei die strukturelle Zone (20) an den Dammbereich angepaßt ist, so ausgelegt, daß die labia majore bedeckt sind.

2. Absorbierender Gegenstand nach Anspruch 1, bei dem der mittlere Teil (60) bei der Verwendung eine im wesentlichen ebene Konfiguration beibehält.

3. Absorbierender Gegenstand nach Anspruch 1, bei dem der absorbierende Kern (40) eine Übergangsschicht (80) und eine Behälterschicht (90) enthält.

4. Absorbierender Gegenstand nach Anspruch 3, bei dem die Behälterschicht (90) Torfmoos ist.

5. Absorbierender Gegenstand nach Anspruch 1, bei dem der absorbierende Gegenstand (5) Befestigungsstreifen (130) aufweist.

6. Absorbierender Gegenstand nach Anspruch 5, bei dem die Befestigungsstreifen (130) an dem absorbierenden Gegenstand (5) an orten zwischen jeder der bevorzugten Biegezonen (50) befestigt sind.

7. Absorbierender Gegenstand nach Anspruch 1, bei dem die strukturelle Zone (120) widerstandsfähig ist gegen das Zusammenfallen bei Nässe in beiden, der X-Richtung, definiert als die Richtung quer zur den Längskanten (100), und der Z-Richtung, definiert als die Richtung normal zu der Abdeckung (10), wobei das Zusammenfallen bei Nässe in der Z-Richtung geringer sein sollte als 10 % bei 1.38 · $10^3$ Pa (0.2 psi) und geringer als 25 % bei 3.45 · $10^3$ Pa (0.5 psi).

8. Absorbierender Gegenstand nach Anspruch 1, bei dem der Abstand zwischen den Biegezonen (60) zwischen 76.2 und 152.4 mm (3 und 6 Zoll) liegt.

**Revendications**

1. Un article absorbant (5) ayant des côtés longitudinaux (100), des extrémités transversales (110), un axe longitudinal, une surface faisant face au corps (20) et une surface faisant face aux vêtements (30), ledit article comprenant :

(a) une enveloppe perméable aux fluides (10) sur ladite surface faisant face au corps;

(b) une partie centrale absorbante (40) en position adjacente par rapport à ladite enveloppe perméable aux fluides (10), ladite partie absorbante (40) ayant une partie centrale (60) située à l'intérieur desdites extrémités transversales (110) et ayant une zone structurelle résiliante qui est résistante à l'effondrement à l'état mouillé ;

(c) ladite partie centrale absorbante (40) comprenant en outre deux zones de courbure préférentielles (50), les zones de courbure préférentielles (50) étant en position transversale par rapport à l'axe longitudinal, et dans laquelle la zone structurelle est située entre les zones de courbure préférentielles ; et

(d) une barrière imperméable aux fluides (30) sur ladite surface faisant face aux vêtements, dans laquelle

(e) ledit article absorbant (5) se courbe de préférence au niveau de ladite zone de courbure préférentielle (50) caractérisé en ce que

(f) on produit la zone de courbure préférentielle (50) en modifiant la densité de l'article absorbant (5) de telle sorte que la partie centrale (60) de l'article absorbant (5) comprennent un matériau ayant une masse de base qui soit supérieure à celle des matériaux à proximité des extrémités transversales (110), et dans lequel ladite zone structurelle (120) soit conforme à la zone périnéale adaptée pour couvrir les grandes lèvres.

2. L'article absorbant selon la revendication 1, dans lequel la partie centrale (60) conserve une configuration essentiellement plane pendant l'utilisation.

3. L'article absorbant selon la revendication 1, dans lequel la partie centrale absorbante (40) contient une couche de transfert (80) et une couche réservoir (90).

4. L'article absorbant selon la revendication 3, dans lequel la couche réservoir est de la sphaigne.

5. L'article absorbant selon la revendication 1, dans laquelle l'article absorbant (5) contient des languettes de fixation (130).

6. L'article absorbant selon la revendication 5, dans lequel les languettes de fixation (130) sont fixées à l'article absorbant (5) à des endroits situés entre chaque zone de courbure préférentielle (50).

7. L'article absorbant selon la revendication 1, dans lequel la zone structurelle (120) qui résiste à l'effondrement à l'état mouillé à la fois dans la direction X définie comme étant la direction transversale aux bords longitudinaux (100), et dans la direction Z qui est définie comme étant la direction normale de la feuille d'enveloppe (10), ce qui a pour résultat que l'effondrement à l'état mouillé dans la direction Z devrait être inférieur à 10% à $1,38 . 10^3$ Pa (0,2 psi) et inférieur à 25% à $3,45 . 10^3$ Pa (0,5 psi).

8. L'article absorbant selon la revendication 1, dans lequel la distance entre lesdites zones de courbure (60) est comprise entre 76,2 et 152,4 mm (3 et 6 pouces).

# FIG.1

# FIG.2

# FIG.3

# FIG. 4

Thigh Shaped Jaws

Mounted Test Element

Compressed Test Element